(19) Europäisches Patentamt
European Patent Office
Office européen des brevets

(11) EP 1 661 887 A1

(12) **EUROPEAN PATENT APPLICATION**

(43) Date of publication:
**31.05.2006 Bulletin 2006/22**

(51) Int Cl.:
***C07D 209/08*** *(2006.01)* ***C07D 401/14*** *(2006.01)*

(21) Application number: **05023338.6**

(22) Date of filing: **26.03.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **27.03.2002 DK 200200480**
**27.03.2002 US 368434 P**

(62) Document number(s) of the earlier application(s) in accordance with Art. 76 EPC:
**03744772.9 / 1 490 353**

(71) Applicant: **H.Lundbeck A/S**
**2500 Valby-Copenhagen (DK)**

(72) Inventors:
- **Zanon, Jacopo**
**30100 Venezia (IT)**
- **Villa, Marco**
**35123 Padova (IT)**
- **Ciardella, Francesco**
**35125 Padova (IT)**

(74) Representative: **Kjerrumgaard, Lars Bo et al**
**H. Lundbeck A/S,**
**Corporate Patents,**
**9, Ottiliavej**
**2500 Valby-Copenhagen (DK)**

Remarks:
This application was filed on 26 - 10 - 2005 as a divisional application to the application mentioned under INID code 62.

(54) **Method for manufacture of sertindole**

(57) The present invention relates to a novel method for manufacture of sertindole comprising manufacturing 5-chloro-1-(4-fluorophenyl)-indole and converting it to sertindole wherein the method for manufacture of 5-chloro-1-(4-fluorophenyl)-indole comprises reacting 5-chloro-indole with a 4-fluorophenylhalide in the presence of a base, a chelating ligand and catalytic amounts of a copper salt comprising copper(I) or copper(II) and an anion which does not interfere in an unfavourable way with the reaction.

EP 1 661 887 A1

## Description

### Field of the invention

The present invention relates to a new method of manufacturing the compound 1-[2-[4-[5-chloro-1-(4-fluorophenyl)-1-H-indol-3-yl]-1-piperidinyi]ethyl]-2-imidazolidinone having the INN name sertindole and a new method of manufacturing the intermediate, 5-chloro-1-(4-fluorophenyl)-indole used in the method.

### Background of the invention

Sertindole is a well-known antipsychotic drug having the formula

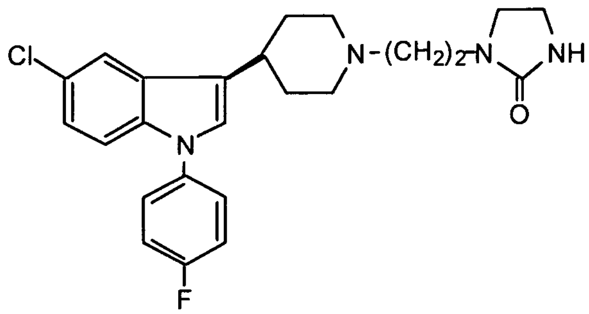

The compound was disclosed in US patent No 4,710,500 and the antipsychotic activity thereof was described in US patent No 5,112,838. Sertindole is a potent centrally acting 5-$HT_2$ receptor antagonist in vivo and has further been disclosed to be active in models indicative of effects in the treatment of anxiety, hypertension, drug abuse and cognitive disorders.

A number of syntheses of sertindole have been disclosed in US patent No 4,710,500 and WO 98/51685. 5-chloro-1-(4-fluorophenyl)-indole is a key intermediate in these syntheses. The syntheses of 5-chloro-1-(4-fluorophenyl)-indole as disclosed in US patent No 4,710,500 and WO 98/51685 require multiple steps from commercially available starting materials, are expensive, occupy production equipment for prolonged periods resulting in low production capacity and result in environmental impact and safety. The synthesis which has been favoured so far for industrial synthesis of sertindole comprises the multiple step synthesis of 5-chloro-1-(4-fluorophenyl)-indole as disclosed in WO 98/51685.

An alternative synthetic strategy for 1-aryl-indoles is the Ullmann arylation of N-unsubstituted indoles with aryl halides catalyzed by large amounts of copper, typically near-stoichiometric amounts or more, as disclosed in e.g. *J.Med.Chem.* **1992,** *35* (6), 1092-1101. The Ullmann arylation has, however, hitherto been disfavoured with regards to the synthesis of 5-chloro-1-(4-fluorophenyl)-indole due to various problems which to those skilled in the art are known to apply to the Ullmann arylation in general as the reactions typically result in moderate yields, around 50%, correspondingly large amounts of coloured by-products and cumbersome work-up procedures caused by the complexation of the reaction product with the copper catalyst. These complexes often require surprisingly harsh treatment to liberate the free reaction product, as known to those skilled in the art.

Hence, there is a desire for new methods for manufacturing of 5-chloro-1-(4-fluorophenyl)-indole. Such new methods may be advantageous in that they are more cost effective, require fewer reaction steps, have reduced impact on the environment, give higher yields, result in increased production capacity, purer crude product and easier work-up procedures.

Recently, Klapars et al. *J.Am.Chem.Soc.* **2001,** 123, 7727-7729, disclosed a variant of the Ullmann arylation wherein copper is present in catalytic amounts together with the chelating ligand trans-1,2-cyclohexanediamine.

### Summary of the invention

It has now surprisingly been found that it is possible to manufacture 5-chloro-1-(4-fluorophenyl)-indole in an efficient way giving good yields by arylation of 5-chloroindole with a 4-fluorophenylhalide in the presence of catalytic amounts of a copper salt and a chelating ligand. This reaction is surprisingly selective. Illustrative of this high selectivity is the fact that there is virtually no by-products formed by reaction between the 5-chloro group of one molecule of 5-chloro-indole and the nitrogen of another molecule of 5-chloro-indole. This type of side reaction would be expected from the disclosure in *J.Am.Chem.Soc.* **2001,** *123,* 7727-7729, which illustrate the reactivity of arylchlorides in this type of reactions. It has even more surprisingly been found that the chelating ligand may be as simple as ethylenediamine. This

reaction gives 5-chloro-1-(4-fluorophenyl)-indole in high yields and purity in a cost-effective single-step synthesis from commercially available starting materials.

Hence, the present invention relates to a novel method for manufacture of sertindole comprising manufacturing 5-chloro-1-(4-fluorophenyl)-indole and converting it to sertindole wherein the method for manufacture of 5-chloro-1-(4-fluorophenyl)-indole comprises reacting 5-chloro-indole with a 4-fluorophenylhalide in the presence of a base, a chelating ligand and catalytic amounts of a copper salt comprising copper(I) or copper(II) and an anion which does not interfere in an unfavourable way with the reaction.

Furthermore, the present invention relates to a method for manufacture of 5-chloro-1-(4-fluorophenyl)-indole comprising reacting 5-chloro-indole with a 4-fluorophenylhalide in the presence of a base, a chelating ligand and catalytic amounts of a copper salt comprising copper(I) or copper(II) and an anion which does not interfere in an unfavourable way with the reaction.

**Detailed description of the invention**

As used throughout the description and the claims, the following definitions apply:

The term '4-fluorophenylhalide' means any compound selected from the group consisting of 4-fluoro-chlorobenzene, 4-fluoro-bromobenzene and 4-fluoro-iodobenzene.

The term 'catalytic amounts' means amounts that are significantly lower than stoichiometric amounts such as less than 20 mol % relative to 5-chloro-indole.

The term 'chelating ligand' means any compound comprising at least two atoms that are able to simultaneously coordinate to the same metal atom.

The term '$C_{1-6}$-alkyl' refers to a branched or unbranched alkyl group having from one to six carbon atoms inclusive, such as methyl, ethyl, 1-propyl, 2-propyl, 1-butyl, 2-butyl, 2-methyl-2-propyl, and 2-methyl-1-propyl.

The term '$C_{1-6}$-alkyl carboxylic acid' refers to $C_{1-6}$-alkyl groups which are terminated by a carboxylic acid.

The term 'aryl' refers to a carbocyclic aromatic group, such as phenyl or naphthyl, in particular phenyl.

In one aspect the present invention relates to a method for manufacture of sertindole comprising manufacturing 5-chloro-1-(4-fluorophenyl)-indole and converting it to sertindole wherein the method for manufacture of 5-chloro-1-(4-fluorophenyl)-indole comprises reacting 5-chloro-indole with a 4-fluorophenylhalide in the presence of a base, a chelating ligand and catalytic amounts of a copper salt comprising copper(I) or copper(II) and an anion which does not interfere in an unfavourable way with the reaction.

In a further aspect, the present invention relates to a method for manufacture of 5-chloro-1-(4-fluorophenyl)-indole comprising reacting 5-chloro-indole with a 4-fluorophenylhalide in the presence of a base, a chelating ligand and catalytic amounts of a copper salt comprising copper(I) or copper(II) and an anion which does not interfere in an unfavourable way with the reaction.

The embodiments described hereafter applies to all aspects of the invention.

In one embodiment of the invention, the chelating ligand is a substituted or unsubstituted 1,10-phenanthrolin, such as an unsubstituted 1,10-phenanthrolin. In another embodiment the chelating ligand is a compound of the formula $X\text{-}(CR^1R^2\text{-}(CR^5R^6)_n\text{-}CR^3R^4\text{-}Y)_m$, wherein X and Y independently are selected from $NR^7R^8$ and $OR^9$, $R^1$-$R^9$ independently are selected from hydrogen, $C_{1-6}$-alkyl, $C_{1-6}$-alkyl carboxylic acid and aryl or one of $R^1$ and $R^2$ together with one of $R^5$ and $R^6$ are $C_{3-6}$-alkylene, m is 1 or 2, and n is 0, 1, 2 or 3. In a preferred embodiment, at least one of X and Y is $NR^7R^8$, and more preferred both of X and Y are $NR^7R^8$. In another preferred embodiment, $R^7$ and $R^8$ are independently selected from hydrogen, $C_{1-6}$-alkyl and $C_{1-6}$-alkyl carboxylic acid, and more preferred $R^7$ and $R^8$ are hydrogen. In yet another preferred embodiment, $R^5$ and $R^6$ are hydrogen. In yet another preferred embodiment, m is 1. In yet another preferred embodiment, n is 0. In yet another preferred embodiment $R^1$ -$R^4$ are hydrogen, or $R^1$ and $R^3$ together are $C_{3-6}$-alkylene and $R^2$ and $R^4$ are hydrogen. Preferred chelating ligands are those selected from the group comprising 1,2-cyclohexanediamine, N,N,N,N-tetramethyl ethylenediamine, N,N-diethyl ethylenediamine, ethylenediamine, ethylenediamine N,N,N,N-tetraacetic acid (EDTA), diethylenetriamine N,N,N,N,N-pentaacetic acid (DTPA) and substituted or unsubstituted 1,10-phenantroline; more preferred chelating ligands are those selected from the group comprising 1,2-cyclohexanediamine, N,N,N,N-tetramethyl ethylenediamine, N,N-diethyl ethylenediamine and ethylenediamine, and the most preferred chelating ligand is ethylenediamine.

In a preferred embodiment of the invention, the 4-fluorophenylhalide is 4-fluoro-bromobenzene or 4-fluoro-iodobenzene as the reactivity of the 4-fluorophenylhalides increases in the order chloro-<bromo-<iodo for this type of reactions. In a preferred embodiment of the invention the 4-fluorophenylhalide is added in a molar surplus relative to 5-chloro-indole. Preferably the molar ratio 4-fluorophenylhalide:5-chloro-indole is in the range from about 1.1 to about 3, more preferred from about 1.2 to about 2.5, and most preferred from about 1.3 to about 2.0.

The methods of manufacture according to the present invention are advantageous as compared to classical Ullmann arylation as they only require catalytic amounts of a copper salt, i.e. less than 20 mol % relative to 5-chloro-indole. Preferably the amount of copper salt is less than 10 mol % relative to 5-chloro-indole and even more preferred

in the range from about 1 to about 5 mol %. The products made according to the present invention may be isolated without the harsh treatment, such as boiling in hydrochloric acid or treatment with cyanides, which often is necessary in order to break the complexes between copper and the product of the classical Ullmann reactions.

Any copper salt comprising copper(I) or copper (II) and an anion which does not interfere in an unfavourable way with the reaction may be applied. Exemplary of anions, which may interfere in an unfavourable way with the reaction, are cynaide, sulphide and selenide. Cyanide may react as a nucleophile and compete with the indole for reaction with the 4-fluorophenylhalide, whereas sulphide and selenide may inactivate the copper catalyst. Those skilled in the art will be aware that other anions also may interfere in an unfavourable way with the reaction and easily realise if an anion interferes in an unfavourable way with the reaction. Preferred copper salts for use in the present invention are selected from the group comprising CuCl, CuBr, CuI, $CuCl_2$, $CuBr_2$, $CuI_2$, $CuOCOCH_3$, $Cu(OCOCH_3)_2$, anhydrous or hydrated $CuSO_4$, $CuCO_3$, $Cu_2O$ and mixtures of said copper salts; more preferred copper salts are those selected from the group comprising CuCl, CuBr, CuI, $CuCl_2$, $CuBr_2$ and $CuI_2$. These work well as catalysts in the reaction and are readily available to reasonable prices. The copper salt may be added in one portion at the start of the reaction or in two or more portions distributed over the reaction time.

Various bases may be employed in the methods of manufacture of the present invention. Exemplary bases are the carbonates, hydrogen carbonates, phosphates, hydrogen phosphates, dihydrogen phosphates, oxides and hydroxides of alkali metals. Preferred bases are potassium and sodium carbonates as these are readily available to a low price and easy to handle. The base is typically present in a molar excess relative to 5-chloro-indole, preferably the amount of base is in the range from about 1.05 molar equivalents to about 2.5 molar equivalents.

The methods of manufacture of the present invention may be performed by heating a neat mixture of the reactants without any solvent or in a suitable solvent system. Exemplary of such solvent systems are toluene, mixtures of toluene and water, ethers such as dioxane, tetrahydrofurane (THF), diethyl ether, dimethyl ether, monoethylene glycol dimethyl ether (monoglyme) and diethylene glycol dimethyl ether (diglyme), amides such as dimethylformamide (DMF), dimethylacetamide (DMA), N-methyl-pyrrolidone (NMP). Preferred solvents are DMF and toluene and most preferred is DMF.

Typically the methods of manufacture of the present invention are performed at temperatures above 80 °C, preferably in the range from 90 °C to 200°C, more preferred in the range from 100°C to 160 °C. Higher yields may be obtained by pretreating the reaction system at a temperature in the range from about 30 °C to about 70 °C, preferably in the range from about 40 °C to about 60 °C, for a period of time ranging from about 0.5 hour to about 20 hours, preferably in the range from about 1 hour to about 15 hours, before completing the reaction at a higher temperature as specified above. Evidently, if the solvent system used is incompatible with the reaction temperature, such as temperatures above 80 °C, then the method may be carried out under pressure.

**Examples**

The following examples is meant to illustrate various embodiments of the invention and should not be read as limiting the scope of protection.

<u>Chromatographic procedures</u>

HPLC and GC analyses were performed according to the procedures described below.

*Analytical Method HPLC - 5-Chloroindole*

| | |
|---|---|
| Instrument HPLC HP 1100 Agilent | Binary Pump Agilent 1100 Series Detector UV Agilent 1100 Series Column Thermostat Agilent 1100 Series Autosampler Agilent 1100 Series Integration Agilent Chemstation |
| Detector | UV 230 nm |
| Column | HP Lichrospher C8 250 x 4 mm, 5 μm |
| Column Temperature | 40 °C |
| Mobile Phase A | Water/Acetonitrile 65:35 |
| Mobile Phase B | Water/Acetonitrile 15:85 |

Table continued

| | | | |
|---|---|---|---|
| Flow | 1.0 mL/min | | |
| Volume injected | 5 μl | | |
| Run time | 45 min | | |
| Gradient | Time | %A | %B |
| | 0 | 100 | 0 |
| | 30 | 0 | 100 |
| | 40 | 0 | 100 |
| | conditioning | | |
| Runtime | 40 min | | |

Assay against external standard

Sample Preparation

Weigh accurately about 50 mg of sample in a 50 mL volumetric flask and add acetonitrile to volume. Transfer 10 mL of obtained solution in a 25 volumetric flask and add acetonitrile to volume. Final concentration 0.2 mg/mL.

Standard Preparation

Weigh accurately about 50 mg of Reference Standard in a 50 mL volumetric flask and add acetonitrile to volume. Transfer 10 mL of obtained solution in a 25 volumetric flask and add acetonitrile to volume. Final concentration 0.2 mg/mL

Analytical Procedure

Inject the Standard three times (at least), integrate the obtained chromatograms and calculate Medium Area. If the Standard Deviation % is less than 1.0% inject the Sample and integrate the chromatogram. Calculate the product assay with the formula:

$$\text{Assay\%} = (\text{Sample Area x Conc. Std x 100})/(\text{Standard Area x Sample Conc.})$$

Where:

Sample Area = Area obtained by sample injection
Standard Area = Average of areas obtained by Standard injection
Sample Conc. = Concentration (mg/ml) of Sample
Standard Conc. = Concentration (mg/ml) of Standard

*Analytical Method HPLC - 5-chloro-1-(4-fluorophenyl)-indole*

Instrument configuration as above except for the gradient.

| | | | |
|---|---|---|---|
| Mobile Phase A | Water/Acetonitrile 65:35 | | |
| Mobile Phase B | Water/Acetonitrile 15:85 | | |
| Run time | 45 min | | |
| Gradient | Time | %A | %A |
| | 0 | 60 | 40 |

Table continued

| Gradient | Time | %A | %A |
|---|---|---|---|
| | 30 | 0 | 100 |
| | 40 | 0 | 100 |
| | conditioning | | |
| Runtime | 40 min | | |

Assay against external standard

Sample Preparation

Weigh accurately about 50 mg of sample in a 50 mL volumetric flask and add acetonitrile to volume. Transfer 10 mL of obtained solution in a 25 volumetric flask and add acetonitrile to volume. Final concentration 0.2 mg/mL.

Standard Preparation

Weigh accurately about 50 mg of Reference Standard in a 50 mL volumetric flask and add acetonitrile to volume. Transfer 10 mL of obtained solution in a 25 volumetric flask and add acetonitrile to volume. Final concentration 0.2 mg/mL.

Analytical Procedure

Inject the Standard three times (at least), integrate the obtained chromatograms and calculate Medium Area. If the Standard Deviation % is less than 1.0% inject the Sample and integrate the chromatogram. Calculate the product assay with the formula:

$$\text{Assay\%} = (\text{Sample Area x Conc. Std x 100})/(\text{Standard Area x Sample Conc.})$$

Where:

Sample Area = Area obtained by sample injection
Standard Area = Average of areas obtained by Standard injection
Sample Conc. = Concentration (mg/ml) of Sample
Standard Conc. = Concentration (mg/ml) of Standard

*Analytical Method GC - 5-chloroindole and 5-chloro-1-(4-fluorophenyl)-indole*

| | |
|---|---|
| Instrument GC | Gc Top 8000 CE Instruments |
| Detector | FID |
| Column | Zebron (ZB-1) |
| | 30 m x 0.25 mm |
| | 0.25 μm |
| Carrier Flow (He) | 1.5 mL/min |
| Split Flow | 50 mL/ml |
| $H_2$ Flow | 30 mL/min |
| Air Flow | 300 mL/min |
| Volume injected | 1 μL |

Table continued

| Run time | 25 min | | |
|---|---|---|---|
| | Step | Temp (°C) | Duration |
| | 1 | 120°C | 3 min |
| | 1→2 | 120°→220°C | 5 min |
| | 2 | 220°C | 20 min |
| | ΔT | 20 °C/min | |
| T inj | 220 °C | | |
| T det | 250 °C | | |

Assay against external standard

Internal Standard Solution

Dilute about 2 ml of Undecane (GC Standards) with Acetone in a 250 mL volumetric flask.

Sample Preparation

Weigh accurately about 250 mg of sample (5-chloroindole or 5-chloro-1-(4-fluorophenyl)-indole) in a 25 mL volumetric flask and add Internal Standard Solution to volume. Final concentration 25 mg/mL.

Standard Preparation

Weigh accurately about 250 mg of Reference Standard (5-chloroindole or 5-chloro-1-(4-fluorophenyl)-indole) in a 25 mL volumetric flask and add Internal Standard Solution to volume. Final concentration 25 mg/mL.

Analytical Procedure

Inject the Standard three times (at least), integrate the obtained chromatograms and calculate the ratio between Area of analyte and Area of Internal Standard. If the ratio Standard Deviation % is less than 1.0% inject the Sample and integrate the chromatogram and calculate the ratio as described above. Calculate the product assay with the formula:

$$\text{Assay\%} = (\text{Sample Area Ratio x Conc. Std x } 100)/(\text{Standard Area Ratio x Sample Conc.})$$

Where:

Sample Area Ratio = Area Ratio obtained by sample injection
Standard Area Ratio = Average of area ratios obtained by Standard injection
Sample Conc. = Concentration (mg/ml) of Sample
Standard Conc. = Concentration (mg/ml) of Standard

*Analytical Method GC - 5-chloro-1-(4-fluorophenyl)-indole - Conversion In-Process-Control*

Instrument configuration as above.

Conversion In-Process-Control

Sample Preparation

Stop the stirring and sample 0.1 mL of reaction solution. Dilute with 5 ml of toluene. Filter the solution obtained and inject.

Calculate the conversion with the formula:

Conversion% = (5-chloro-1-(4-fluorophenyl)-indole Area x 100)/(5-Chloroindole Area + 5-chloro-1-(4-fluorophenyl)-indole Area)

Where:

5-chloro-1-(4-fluorophenyl)-indole Area = Area detected for 5-chloro-1-(4-fluorophenyl)-indole
5-Chloroindole Area = Area detected for 5-Chloroindole

Identification of product

NMR spectra were determined on a Bruker Avance 300 spectrometer
$^1$H-NMR $CDCl_3$ 300MHz (δ ppm, *J*Hz): 7.70 (1H, d, *J*= 2.0); 7.49-7.39 (3H, m); 7.32 (1H, d, *J* = 3.2); 7.30-7.17 (3H, m); 6.66 (1H, d, *J*= 3.2).
$^{13}$C-NMR $CDCl_3$ 75MHz (δ ppm, $J_{C,F}$ Hz): 161.68 (d, $J_{C,F}$ = 245.0); 135.87 (d, $J_{C,F}$ = 2.0); 134.96; 130.62; 129.75; 126.59 (d, $J_{C,F}$ = 8.3); 126.49; 123.18; 120.97; 117.04 (d, $J_{C,F}$ = 22.0); 111.71; 103.59.
$^{19}$F-NMR $CDCl_3$ 282MHz (δ ppm): 114.94 (m).

These data are in agreement with the structure of 5-chloro-1-(4-fluorophenyl)-indole.

Synthetic examples with toluene as solvent

*Example 1: N,N,N,N tetramethyl ethylenediamine as ligand*

A jacketed glass reactor was charged with 40 g of crude 5-chloro-indole (80% pure as determined by HPLC) (32 g, 0.211 mol), $K_2CO_3$ (40.2 g, 0.2902 mol), 4-fluoro-bromobenzene (92.3 g, 0.5277 mol), CuI (2.5 g, $1.32{*}10^{-2}$ mol), N,N,N,N-tetramethyl ethylenediamine (3.2 g, $5.28{*}10^{-2}$ mol) and 80 mL of toluene. The mixture was heated to reflux (about 115 °C), under vigorous stirring, and maintained for 40 hours.

After cooling to 60 °C, 80 mL of Toluene and 80 mL of water were added and the mixture was maintained under stirring at 50 °C for ½ hour and the organic layer was separated and treated with 80 mL of water. The residual carbonates were then dissolved by slow addition of aqueous HCl 32% until solution reached pH = 2-3. The mixture was maintained under stirring at 50 °C for ½ hour the aqueous layers were eliminated. The organic layer was then concentrated, by solvent distillation at reduced pressure, and the crude product was obtained as an oil (47.2 g). The yield, based on HPLC (assay against ext. Std.), was about 42%.

*Example 2: N,N diethyl ethylenediamine as ligand*

Following the procedure of example 1 except that N,N-diethyl ethylenediamine was used in stead of N,N,N,N-tetramethyl ethylenediamine the crude product was obtained as an oil (84 g). The yield, based on HPLC (assay against ext. Std.), was about 50%.

*Example 3: Trans-1,2-cyclohexanediamine as ligand*

A jacketed glass reactor was charged with 10 g of crude 5-chloro-indole (80% pure as determined by HPLC) (8 g, $5.2{*}10^{-2}$ mol), $K_2CO_3$ (12.7 g, $9.2{*}10^{-2}$ mol), 4-fluoro-bromobenzene (12.7 g, $7.3{*}10^{-2}$ mol), CuI (1.26 g, $6.6{*}10^{-3}$ mol), *trans*-1,2-cyclohexanediamine (1.13 g, $9.9{*}10^{-3}$ mol) and 20 mL of toluene. The mixture was heated to reflux (about 115 °C), under vigorous stirring, and maintained for 12 hours. The conversion checked by GC was about 79%.

After cooling to 60 °C, the solid residual were filtered off and the organic solution was then concentrated, by solvent distillation at reduced pressure, and the crude product was obtained as an oil (15.4 g)

*Example 4: $K_3PO_4$ as base*

A jacketed glass reactor was charged with 20 g of crude 5-chloro-indole (80% pure as determined by HPLC) (16 g, 0.106 mol), $K_3PO_4$ (18.6 g, 0.088 mol), 4-fluoro-bromobenzene (46.2 g, 0.263 mol), CuI (1.25 g, $1.32{*}10^{-2}$ mol), ethylenediamine (1.58 g, $2.62{*}10^{-2}$ mol) and 40 mL of toluene. The mixture was heated to reflux (about 115°C), under vigorous stirring, and maintained for 22 hours. An additional amount of $K_3PO_4$ (9.3 g, $4.4{*}10^{-2}$ mol) was added and the

mixture was stirred for 19h. The conversion checked by GC was about 42%.

After cooling to 60 °C, 80 mL of Toluene and 80 mL of water were added and the mixture was maintained under stirring at 50 °C for ½ hour and the organic layer were separated and treated with 80 mL of water. The residual phosphates were then dissolved by slow addition of aqueous HCl 32% until solution reached pH = 2-3. The mixture was maintained under stirring at 50 °C for ½ hour the aqueous layers were eliminated. The organic layer was then concentrated, by solvent distillation at reduced pressure, and the crude product was obtained as an oil (62.3 g).

*Example 5: CuBr as catalyst source*

A jacketed glass reactor was charged with 40 g of crude 5-chloro-indole (80% pure as determined by HPLC) (32 g, 0.211 mol), $K_2CO_3$ (40.2 g, 0.2902 mol), 4-fluoro-bromobenzene (92.3 g, 0.5277 mol), CuBr (1.89 g, $1.32{*}10^{-2}$ mol), ethylenediamine (3.2 g, $5.28{*}10^{-2}$ mol) and 80 ml of toluene. The mixture was heated to reflux (about 115 °C), under vigorous stirring, and maintained for 32 hours. The conversion checked by GC was about 92%.

After cooling to 60 °C, 80 mL of toluene and 80 mL of water were added and the mixture was maintained under stirring at 50 °C for ½ hour and the organic layer was separated and treated with 80 mL of water. The residual carbonates were then dissolved by slow addition of aqueous HCl 32% until solution reached pH = 2-3. The mixture was maintained under stirring at 50 °C for ½ hour the aqueous layers were eliminated. The organic layer was then concentrated, by solvent distillation at reduced pressure, and the crude product was obtained as an oil (64.4 g).

*Example 6: CuCl as catalyst source*

A jacketed glass reactor was charged with 40 g of crude 5-chloro-indole (80% pure as determined by HPLC) (32 g, 0.211 mol), $K_2CO_3$ (40.2 g, 0.2902 mol), 4-fluoro-bromobenzene (92.3 g, 0.5277 mol), CuCl (1.31 g, $1.32{*}10^{-2}$ mol), ethylenediamine (3.2 g, $5.28{*}10^{-2}$ mol, 25%) and 80 mL of toluene. The mixture was heated to reflux (about 115°C), under vigorous stirring, and maintained for 32 hours. The conversion checked by GC was about 92%.

After cooling to 60 °C, 80 mL of toluene and 80 mL of water were added and the mixture was maintained under stirring at 50 °C for ½ hour and the organic layer was separated and treated with 80 mL of water. The residual carbonates were then dissolved by slow addition of aqueous HCl 32% until solution reached pH = 2-3.The mixture was maintained under stirring at 50°C for ½ hour the aqueous layers were eliminated. The organic layer was then concentrated, by solvent distillation at reduced pressure, and the crude product was obtained as an oil (7.81g).

*Example 7: $CuBr_2$ as catalyst source*

A glass jacketed reactor was charged with 20 g of crude 5-chloro-indole (80% pure as determined by HPLC) (16 g, 0.106 mol), $K_2CO_3$ (20 g, 0.144 mol), 4-fluoro-bromobenzene (46.1 g, 0.26 mol), $CuBr_2$ (1.46 g, $6.6{*}10^{-3}$ mol), ethylenediamine (1.58 g, $2.6{*}10^{-2}$ mol) and 40 ml of toluene. The mixture was heated to reflux (about 115 °C), under vigorous stirring, and maintained for 28 hours. The conversion checked by GC was about 44% (after 20 hours the conversion checked by GC was about 43%).

After cooling to 60 °C, 50 mL of Toluene and 40 mL of water were added and the mixture was cooled to 50 °C under stirring. The residual carbonate were then dissolved by slow addition of aqueous HCl 32% until solution reached pH = 2-3. The mixture was maintained under stirring at 50 °C for ½ hour before the organic layer was separated. The organic layer was treated several times with saturated solution of Sodium Chloride and water under stirring at 50 °C and concentrated, by solvent distillation at reduced pressure. The crude product was obtained as an oil (41 g)

Examples 8-18 illustrate variations of the CuI-Ethylenediamine-$K_2CO_3$-toluene system. They were performed according to the procedure of example 1 except for the details specified. The amounts are given relative to the amount of 5-chloro-indole (calculated as pure 5-chloro-indole). % means mol %, equivalent means molar equivalent, and volume means ml of solvent per g of 5-chloro-indole.

*Example 8:*

10% of CuI, 15% of ethylenediamine, 2.1 equivalent of $K_2CO_3$, 1.1 equivalent of 4-fluoro-bromobenzene, 2 volumes of toluene, 16h reflux. The conversion checked by GC was about 99.5%.

*Example 9:*

1% of CuI, 5% of ethylenediamine, 1.5 equivalent of $K_2CO_3$, 1.1 equivalent of 4-fluoro-bromobenzene, 2 volumes of toluene, 10h reflux. The conversion checked by GC was about 52%.

*Example 10:*

1% of CuI, 5% of ethylenediamine, 1.5 equivalent of $K_2CO_3$, 1.3 equivalent of 4-fluoro-bromobenzene, 2 volumes of toluene, 10h reflux. The conversion checked by GC was about 45%.

*Example 11:*

5% of CuI, 15% of ethylenediamine, 1.05 equivalent of $K_2CO_3$, 1.2 equivalent of 4-fluoro-bromobenzene, 2 volumes of toluene, 18h distilling off water as azeotrope and recycling toluene. The conversion checked by GC was about 55%.

*Example 12:*

5% of CuI, 15% of ethylenediamine, 2.1 equivalent of $K_2CO_3$, 1.1 equivalent of 4-fluoro-bromobenzene, 2 volumes of toluene, 36h reflux. The conversion checked by GC was about 96%.

*Example 13:*

5% of CuI, 15% of ethylenediamine, 1.5 equivalent of $K_2CO_3$, 1.1 equivalent of 4-fluoro-bromobenzene, 2 volumes of toluene, 36h reflux. The conversion checked by GC was about 95%.

*Example 14:*

5% of CuI, 20% of ethylenediamine, 1.1 equivalent of $K_2CO_3$, 1.1 equivalent of 4-fluoro-bromobenzene, 2 volumes of Toluene, 44h reflux. The conversion checked by GC was about 99%.

*Example 15:*

5% of CuI, 20% of ethylenediamine, 1.1 equivalent of $K_2CO_3$, 2 equivalent of 4-fluoro-bromobenzene, 2 volumes of toluene, 36h reflux. Addition of CuI in two portions (2x2.5%, 2$^{nd}$ after 10 h refluxing). The conversion checked by GC was about 98%.

*Example 16:*

5% of CuI, 1.14 equivalent of ethylenediamine, 1.1 equivalent of $K_2CO_3$, 2 equivalent of 4-fluoro-bromobenzene, 2 volumes of toluene, 24h reflux. The conversion checked by GC was about 86%.

*Example 17:*

2.5% of CuI, 40% of ethylenediamine, 1.1 equivalent of $K_2CO_3$, 2 equivalent of 4-fluoro-bromobenzene, 2 volumes of toluene, 26h reflux. The conversion checked by GC was about 87%.

*Example 18: Under moderate pressure*

5% of CuI, 20% of ethylenediamine, 1.1 equivalent of $K_2CO_3$, 2 equivalent of 4-fluoro-bromobenzene, 2 volumes of toluene. The reaction mixture was heated to 120 °C in a closed reactor for 44 h allowing the pressure to increase to a maximum of 2 bar. The conversion checked by GC was about 87%.

<u>Toluene and water as solvent system</u>

*Example 19: $K_3PO_4$ as base*

A jacketed glass reactor was charged with 40 g of crude 5-chloro-indole (80% pure as determined by HPLC) (32 g, 0.211 mol), $K_3PO_4$ (56 g, 0.264 mol), 4-fluoro-bromobenzene (92.3 g, 0.5277 mol), CuI (2.5 g, $1.32*10^{-2}$ mol), ethylenediamine (3.2 g, $5.28.10^{-2}$ mol), 80 mL of toluene and 20 ml of water. The mixture was heated to reflux (about 115 °C), under vigorous stirring, and maintained for 40 hours. The conversion checked by GC was about 89%.

After cooling to 60 °C, 80 mL of Toluene and 80 mL of water were added and the mixture was maintained under stirring at 50 °C for ½ hour and the organic layer was separated and treated with 80 mL of water. The residual phosphates

were then dissolved by slow addition of aqueous HCl 32% until solution reached pH = 2-3. The mixture was maintained under stirring at 50 °C for ½ hour the aqueous layers were eliminated. The organic layer was then concentrated, by solvent distillation at reduced pressure, and the crude product was obtained as an oil (86.4 g).

*Example 20: $K_2CO_3$ as base*

A jacketed glass reactor was charged with 40 g of crude 5-chloro-indole (80% pure as determined by HPLC) (32 g, 0.211 mol), $K_2CO_3$ (40.2 g, 0.290 mol), 4-fluoro-bromobenzene (92.3 g, 0.5277 mol), CuI (2.5 g, $1.32{*}10^{-2}$ mol), ethylenediamine (3.2 g, $5.28{*}10^{-2}$ mol), 80 ml of toluene and 20 mL of water. The mixture was heated to reflux (about 110 °C), under vigorous stirring, and maintained for 36 hours. The conversion checked by GC was about 67%.

After cooling to 60 °C, 80 mL of toluene and 80 mL of water were added and the mixture was maintained under stirring at 50 °C for ½ hour and the organic layer were separated and treated with 80 mL of water. The residual carbonates were then dissolved by slow addition of aqueous HCl 32% until solution reached pH = 2-3. The mixture was maintained under stirring at 50 °C for ½ hour the aqueous layers were eliminated. The organic layer was then concentrated, by solvent distillation at reduced pressure, and the crude product was obtained as an oil (68 g). The yield, based on HPLC (assay against ext. Std.), was about 50%.

Dimethylformamide (DMF) as a solvent

*Example 21:*

A glass jacketed reactor was charged, under nitrogen, with distilled 5-chloro-indole (94% pure as determined by HPLC) (200 g, 1.32 mol), $K_2CO_3$ (200 g, 1.45 mol), 4-fluoro-bromobenzene (461 g, 2.63 mol), CuI (12.6 g, 0.066 mol), ethylenediamine (15.9 g, 0.26 mol) and 400 mL of dimethylformamide. The mixture was heated to 40°C under vigorous stirring and kept at that temperature for 12 hours whereafter the mixture was to reflux (about 130-135°C), under vigorous stirring, by increasing the jacket temperature over period of 45 minutes to 145 °C and maintained at reflux for 5 hours.

After cooling to 60 °C, 400 mL of toluene and 400 mL of water were added and the mixture was cooled to 50 °C under stirring. The organic phase was separated and washed, at 50 °C with diluted hydrochloric acid (5 ml HCl 32% + 100 ml $H_2O$) and finally with a solution of diluted ammonia (5 mL of $NH_3$ 33% + 200 mL of $H_2O$). The solvent was then removed by distillation at reduced pressure and the crude product was obtained as an oil (469 g). The yield, based on HPLC (assay against ext. Std.), was about 94%.

*Example 22: CuBr as catalyst source*

A glass jacketed reactor was charged with 20 g of crude 5-chloro-indole (80% pure as determined by HPLC) (16 g, 0.106 mol), $K_2CO_3$ (20 g, 0.144 mol), 4-fluoro-bromobenzene (47.7 g, 0.27 mol), CuBr (0.95 g, $6.6{*}\ 10^{-3}$ mol), ethylenediamine (1.58 g, $2.6{*}10^{-2}$ mol) and 40 mL of dimethylformamide. The mixture was heated to reflux (about 130-135 °C), under vigorous stirring, and maintained for 20 hours. The conversion checked by GC was about 99.5% (after 6 hours the conversion checked by GC was about 81%).

After cooling to 60 °C, 80 mL of Toluene and 40 mL of water were added and the mixture was cooled to 50 °C under stirring. The residual carbonate were then dissolved by slow addition of aqueous HCl 32% until solution reached pH = 2-3. The mixture was maintained under stirring at 50 °C for ½hour. The organic layer was separated and treated with 40 mL of water. The mixture was maintained under stirring at 50 °C for ½ hour the aqueous layers were eliminated. The organic layer was treated several times with saturated solution of ammonium sulphate and water under stirring at 50 °C and then concentrated by solvent distillation at reduced pressure. The crude product was obtained as an oil (38.4 g). The yield, based on HPLC (assay against ext. Std.), was about 80%.

*Example 23: CuCl and KI as catalyst source*

A glass jacketed reactor was charged with 20 g of crude 5-chloro-indole (80% pure as determined by HPLC) (16 g, 0.106 mol), $K_2CO_3$ (20 g, 0.144 mol), 4-fluoro-bromobenzene (47.7 g, 0.27 mol), CuCl (0.595 g, $6.0{*}10^{-3}$ mol), ethylenediamine (1.58 g, $2.6{*}10^{-2}$ mol) and 40 mL of dimethylformamide. The mixture was heated to reflux (about 130-135 °C), under vigorous stirring. After 4 hours was added KI (1.16 g, $6.99{*}10^{-3}$ mol). The mixture was then maintained at reflux for 16 h. The conversion checked by GC was about 99.5% (after 6 hours the conversion checked by GC was about 53%).

After cooling to 60 °C, 80 mLof Toluene and 40 mL of water were added and the mixture was cooled to 50 °C under stirring. The residual carbonates were then dissolved by slow addition of aqueous HCl 32% until solution reached

pH = 2-3. The mixture was maintained under stirring at 50 °C for ½ hour the organic layer were separated and treated with 40 mL of water. The mixture was maintained under stirring at 50 °C for ½ hour the aqueous layers were eliminated. The organic layer was treated several times with saturated solution of ammonium sulfate and water under stirring at 50 °C then and concentrated, by solvent distillation at reduced pressure. The crude product was obtained as an oil (37.5 g). The yield, based on HPLC (assay against ext. Std.), was about 82%.

Examples 24-29 illustrate variations of the CuI-Ethylenediamine-$K_2CO_3$-Dimethylformamide system. They were performed according to the procedure of example 21 except for the scale which was 40 g of 5-chloro-indole and the details specified. The amounts are given relative to the amount of 5-chloro-indole (calculated as pure 5-chloro-indole). % means mol %, equivalent means molar equivalent, and volume means ml of solvent per g of 5-chloro-indole.

*Example 24*

5% of CuI, 20% of ethylenediamine, 1.1 mol of $K_2CO_3$, 2 mol of 4-fluoro-bromobenzene, 2 volumes of dimethylformamide, 29h 120°C. The conversion checked by GC was about 80%.

*Example 25*

5% of CuI, 20% of ethylenediamine, 1.1 mol of $K_2CO_3$, 2 mol of 4-fluoro-bromobenzene, 2 volumes of dimethylformamide, 6h 135°C. The conversion checked by GC was about 99%.

*Example 26*

5% of CuI, 20% of ethylenediamine, 1.1 mol of $K_2CO_3$, 1.2 mol of 4-fluoro-bromobenzene, 2 volumes of dimethylformamide. Pretreatment of catalytic system 1 h at 50°C. Reaction 5.5h 135°C. The conversion checked by GC was about 94%.

*Example 27*

5% of CuI, 20% of ethylenediamine, 1.1 mol of $K_2CO_3$, 2 mol of 4-fluoro-bromobenzene, 2 volumes of dimethylformamide and 0.5 volumes of water. Pretreatment of catalytic system 1 h at 50 °C. Reaction 19h 118 °C (reflux). The conversion checked by GC was about 58%.

*Example 28*

5% of CuI, 20% of ethylenediamine, 1.1 mol of $K_2CO_3$, 2 mol of 4-fluoro-bromobenzene, 2 volumes of Dimethylformamide. Pretreatment of catalytic system 14 h at 50 °C. Reaction 7h 135 °C. The conversion checked by GC was about 92.2%.

*Example 29*

5% of CuI, 20% of ethylenediamine, 1.1 mol of $K_2CO_3$, 2 mol of 4-fluoro-bromobenzene, 2 volumes of dimethylformamide. NO Pretreatment of catalytic system 50 °C. Reaction 7h 135 °C. The conversion checked by GC was about 78%.

Example 30 illustrates the removal of the impurity 5-bromo-1-(4-fluorophenyl)-indole, which is generated in levels up to 1% by performing a halogen exchange during work-up. Lowering of the impurity by recrystallisation turned out to be difficult.

*Example 30*

A glass jacketed reactor was charged, under nitrogen, with 5-chloroindole (200 g, 1.32 mol), $K_2CO_3$ (200 g, 1.45 mol), 4-bromo-fluorobenzene (347 g, 1.98 mol) and 400 ml of dimethylformamide. The mixture was heated to 50°C and ethylenediamine (16 g, 0.26 mol) and CuI (12.5 g, 0.066 mol) were charged in the reactor. The mixture was kept at that temperature for 1.5 hours, then was heated up to 130°C for 1 hour and finally was heated to reflux temperature (about 139°C) for 4 hours. The conversion checked by HPLC was >95%. When the coupling reaction was completed (ref. Example.doc), the mixture was cooled to 100°C and 800ml of toluene were added. After cooling to 60°C the mixture was washed with a solution of diluted ammonia (80 ml of $NH_3$ 30% + 400 ml of $H_2O$). The organic phase was washed at 40°C with diluted hydrochloric acid (50 ml of HCl 32% + 200 ml of $H_2O$) and finally with diluted ammonia (44 ml of $NH_3$ 30% + 300 ml of water). The organic solution was concentrated by distillation at normal pressure and then at reduced

pressure by stripping with 1-methyl-2-pyrrolidinone (NMP). The residue was diluted with NMP. CuCl (17-35 g, 0.17-0.35 mol) and CuI (2.5g, 0.013 mol) were charged in the reactor and mixture was heated up to 140°C for 6 hours. After diluting with toluene (600 ml), the mixture was filtered and then washed with ammonia (45 ml of $NH_3$ 30% + 300 ml of $H_2O$). The organic phase was concentrated by distillation at normal pressure, then was diluted with sulfolane and concentrated under vacuum. The crude was finally purified by thin film distillation.

Dioxane as solvent

*Example 31: Trans-1,2-Cyclohexanediamine as ligand*

A jacketed glass reactor was charged with 5g of crude 5-chloro-indole (80% pure as determined by HPLC) (4 g, $2.6{*}10^{-2}$ mol), $K_2CO_3$ (9.58 g, $6.9{*}10^{-2}$ mol), 4-fluoro-bromobenzene (6.34 g, $3.6{*}10^{-2}$ mol), CuI (0.063 g, $6.6{*}10^{-4}$ mol), *trans*-1,2-cyclohexanediamine (0.377 g, $3.3{*}10^{-3}$ mol) and 33 mL of dioxane. The mixture was heated to about 110 °C, under vigorous stirring, and maintained for 25 hours. The conversion checked by GC was about 45%.

After cooling to 60 °C, the solid residual were filtered off and the organic solution was then concentrated, by solvent distillation at reduced pressure, and the crude product was obtained as an oil (8.2 g).

Neat - Without Solvent

*Example 32:*

A jacketed glass reactor was charged with 30 g of distilled 5-Cl-indole (96% pure as determined by HPLC) (28.8 g, 0.190 moles), $K_2CO_3$ (30.1 g, 0.218 moles), 4-fluoro-bromobenzene (143.4 g, 0.819 moles), CuI (1.88 g, $9.89{*}10^{-3}$ moles) and ethylenediamine (2.38 g, $3.96{*}10^{-2}$ moles). The mixture was heated to 130-135 °C under vigorous stirring, and maintained for 5 hours.

After cooling to 50 °C, 80 mL of Toluene and 80 mL of water were added and the mixture was maintained under stirring at 50 °C for 15 minutes. The residual carbonates were then dissolved by slow addition of $H_2SO_4$ 36% until solution reached pH = 2-3 (about 40 mL). The mixture was maintained under stirring at 50 °C for ½ hour then cooled to room temperature and stirred overnight. The aqueous layer (upper phase) was eliminated. The organic phase was washed two times with water (2x50mL) and then concentrated, by solvent distillation at reduced pressure. The crude product was obtained as an oil (115.9 g). The yield, based on HPLC (assay against ext. Std.), was about 42%.

## Claims

**1.** Method for manufacture of sertindole comprising manufacturing 5-chloro-1-(4-fluorophenyl)-indole and converting it to sertindole **characterised in that** the method for manufacture of 5-chloro-1-(4-fluorophenyl)-indole comprises reacting 5-chloro-indole with a 4-fluorophenylhalide in the presence of a base, a chelating ligand and catalytic amounts of a copper salt comprising copper(I) or copper(II) and an anion which does not interfere in an unfavourable way with the reaction.

**2.** Method for manufacture of 5-chloro-1-(4-fluorophenyl)-indole comprising reacting 5-chloro-indole with a 4-fluorophenylhalide in the presence of a base, a chelating ligand and catalytic amounts of a copper salt comprising copper (I) or copper(II) and an anion which does not interfere in an unfavourable way with the reaction.

**3.** Method according to claim 1 or 2 **characterised in that** the chelating ligand is a substituted or unsubstituted 1,10-phenanthrolin or a compound of the formula $X\text{-}(CR^1R^2\text{-}(CR^5R^6)n\text{-}CR^3R^4\text{-}Y)_m$, wherein X and Y independently are selected from $NR^7R^8$ and $OR^9$, $R^1$-$R^9$ independently are selected from hydrogen, $C_{1-6}$-alkyl, $C_{1-6}$-alkyl carboxylic acid and aryl or one of $R^1$ and $R^2$ together with one of $R^5$ and $R^6$ are $C_{3-6}$-alkylene, m is 1 or 2 and n is 0, 1, 2 or 3.

**4.** Method according to claim 3 **characterised in that** the chelating ligand is selected from the group comprising 1,2-cyclohexanediamine, N,N,N,N-tetramethyl ethylenediamine, N,N-diethyl ethylenediamine, ethylenediamine, ethylenediamine N,N,N,N-tetraacetic acid (EDTA), diethylenetriamine N,N,N,N,N-pentaacetic acid (DTPA) and substituted or unsubstituted 1,10-phenantroline, typically the chelating ligand is selected from 1,2-cyclohexanediamine, N,N,N,N-tetramethyl ethylenediamine, N,N-diethyl ethylenediamine and ethylenediamine, in particular the chelating ligand is ethylenediamine.

**5.** Method according to any one of claims 1-4 **characterised in that** the copper salt is selected from CuCl, CuBr, CuI,

$CuCl_2$, $CuBr_2$, $CuI_2$, $CuOCOCH_3$, $Cu(OCOCH_3)_2$, anhydrous or hydrated $CuSO_4$, $CuCO_3$, $Cu_2O$ and mixtures of said copper salts, typically the copper salt is selected from CuCl, CuBr, CuI, $CuCl_2$, $CuBr_2$ or $CuI_2$.

6. Method according to any one of claims 1-5 **characterised in that** the 4-fluorophenylhalide is selected from 4-fluoro-bromobenzene or 4-fluoro-iodobenzene, such as 4-fluoro-bromobenzene.

7. Method according to any one of claims 1-6 **characterised in that** the 4-fluorophenylhalide is added in a molar surplus relative to the 5-chloro-indole.

8. Method according to claim 7 **characterised in that** the molar surplus is in the range from 1.1 to 3.

9. Method according to any one of claims 1-8 **characterised in that** the catalytic amounts of the copper salt is less than 20 mol % relative to the 5-chloro-indole, typically less than 10 mol % relative to the 5-chloro-indole, such as in the range from about 1 to about 5 mol %.

10. Method according to any one of claims 1-9 **characterised in that** the base is selected from the carbonates, hydrogen carbonates, phosphates, hydrogen phosphates, dihydrogen phosphates, oxides and hydroxides of alkali metals.

11. Method according to claim 10 **characterised in that** the base is present in a molar excess relative to the 5-chloro-indole, typically the amount of base is in the range from about 1.05 molar equivalents to about 2.5 molar equivalents.

12. Method according to any one of claims 1-11 **characterised in that** reaction is completed at temperatures in the range from above 80 °C to 200 °C, typically in the range from 100 °C to 160 °C.

European Patent Office

# EUROPEAN SEARCH REPORT

Application Number
EP 05 02 3338

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | ARTIS KLAPARS ET AL: "A General and Efficient Copper Catalyst for the Amidation of Aryl Halides and the N-Arylation of Nitrogen Heterocycles" JOURNAL OF THE AMERICAN CHEMICAL SOCIETY, AMERICAN CHEMICAL SOCIETY, WASHINGTON, DC, US, vol. 123, 7 December 2001 (2001-12-07), pages 7727-7729, XP002211988 ISSN: 0002-7863 * examples * | 1-12 | C07D209/08<br>C07D401/14 |
| Y | KANG S-K ET AL: "COPPER-CATALYZED N-ARYLATION OF ARYL IODIDES WITH BENZAMIDES OR NITROGEN HETEROCYLCES IN THE PRESENCE OF ETHYLENEDIAMINE" SYNLETT, THIEME INTERNATIONAL, STUTTGART, DE, vol. 3, 4 March 2002 (2002-03-04), pages 427-430, XP001156275 ISSN: 0936-5214 * examples * | 1-12 | TECHNICAL FIELDS SEARCHED (IPC) |
| Y,D | PERREGAARD J ET AL: "NONCATALEPTOGENIC, CENTRALLY ACTING DOPAMINE D-2 AND SEROTONIN 5-HT2 ANTOGONISTS WITHIN A SERIES OF 3-SIBSTITUTED 1-(4-FLUOROPHENYL)-1H-INDOLES" 20 March 1992 (1992-03-20), JOURNAL OF MEDICINAL CHEMISTRY, AMERICAN CHEMICAL SOCIETY. WASHINGTON, US, PAGE(S) 1092-1101 , XP000568758 ISSN: 0022-2623 * examples * | 1-12 | C07D |
| A,D | US 4 710 500 A (PERREGAARD ET AL) 1 December 1987 (1987-12-01) * example 2 * | 1-12 | |

The present search report has been drawn up for all claims

| Place of search | Date of completion of the search | Examiner |
|---|---|---|
| Munich | 6 December 2005 | Menegaki, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

## ANNEX TO THE EUROPEAN SEARCH REPORT ON EUROPEAN PATENT APPLICATION NO. EP 05 02 3338

This annex lists the patent family members relating to the patent documents cited in the above-mentioned European search report.
The members are as contained in the European Patent Office EDP file on
The European Patent Office is in no way liable for these particulars which are merely given for the purpose of information.

06-12-2005

| Patent document cited in search report | Publication date | Patent family member(s) | Publication date |
|---|---|---|---|
| US 4710500 A | 01-12-1987 | AU 583607 B2 | 04-05-1989 |
| | | CA 1256437 A1 | 27-06-1989 |
| | | CA 1338327 B | 14-05-1996 |
| | | DK 157686 A | 11-10-1986 |
| | | DK 169674 B1 | 09-01-1995 |
| | | EP 0200322 A1 | 05-11-1986 |
| | | FI 861505 A | 11-10-1986 |
| | | HK 116793 A | 05-11-1993 |
| | | IE 58370 B1 | 08-09-1993 |
| | | JP 1885645 C | 22-11-1994 |
| | | JP 6004587 B | 19-01-1994 |
| | | JP 61236764 A | 22-10-1986 |
| | | LU 90218 A9 | 27-04-1998 |
| | | NL 970016 I1 | 02-06-1997 |
| | | US RE34299 E | 29-06-1993 |

EPO FORM P0459

For more details about this annex : see Official Journal of the European Patent Office, No. 12/82